Europäisches Patentamt

European Patent Office

Office européen des brevets

(11) Veröffentlichungsnummer: **0 346 836**
**A2**

(12) **EUROPÄISCHE PATENTANMELDUNG**

(21) Anmeldenummer: **89110697.3**

(22) Anmeldetag: **13.06.89**

(51) Int. Cl.4: **C07D 307/50 , D21C 1/02 , D21C 3/02 , D21C 3/20**

(30) Priorität: **13.06.88 CS 4060/88**

(43) Veröffentlichungstag der Anmeldung:
**20.12.89 Patentblatt 89/51**

(84) Benannte Vertragsstaaten:
**AT DE FR IT SE**

(71) Anmelder: **JEDNOTNE ZEMEDELSKE DRUZSTVO JANA CERNEHO SE SIDLEM**

**691 55 v Moravské Nové Vsi(CS)**

(72) Erfinder: **Seifert, Reiner, Dipl.-Ing.**
**Kurkova 1209**
**Praha 8(CS)**
Erfinder: **Kratochvil, Zdenek**
**Chmelova 9**
**Praha 10(CS)**

Erfinder: **Ditl, Pavel, Dipl.-Ing.**
**Pivonková 22**
**Praha 10(CS)**
Erfinder: **Pribyl, Oldrich, Dipl.-Ing.**
**Dimitrovovo nám. 27**
**Praha 7(CS)**
Erfinder: **Kühnel, Egon**
**Heinrichova 4**
**Brno(CS)**
Erfinder: **Schneider, Ales, Dipl.-Ing.**
**Socharská 3**
**Praha 7(CS)**

(74) Vertreter: **Patentanwälte Beetz sen. - Beetz jun. Timpe - Siegfried - Schmitt-Fumian-Mayr**
**Steinsdorfstrasse 10**
**D-8000 München 22(DE)**

(54) **Verfahren und Vorrichtung zur kontinuierlichen Herstellung von 2-Furaldehyd, Cellulose und Lignin aus Lignocellulosematerialien.**

(57) Die Erfindung betrifft ein abfallfrei und ohne Verwendung von Chemikalien arbeitendes Verfahren sowie eine Vorrichtung zur kontinuierlichen Herstellung von 2-Furaldehyd, Cellulose und Lignin aus Lignocellulosematerialien, die zu einer hohen Ausbeute an diesen Substanzen führen. Das Verfahren beruht auf einer zweistufigen kontinuierlichen Hydrolyse von desintegriertem Lignocellulosematerial unter Druck in einem ersten kontinuierlichen Hydrolysereaktor (5) sowie einem nachgeschalteten zweiten kontinuierlichen Hydrolysereaktor (7), in dem eine zweistufige Hydrolyse durchgeführt wird; die resultierende Suspension wird nach Abpressen der flüssigen Phase, die den Hauptgehalt an 2-Furaldehyd enthält, in einer Entspannungsvorrichtung (19) auf Atmosphärendruck entspannt und in einer Extraktionsvorrichtung (22) extrahiert, wobei Lignin und Cellulose gewonnen werden. Der 2-Furaldehyd wird aus der flüssigen Phase durch zweistufige Rektifikation unter Atmosphärendruck gewonnenen.

## Verfahren und Vorrichtung zur kontinuierlichen Herstellung von 2-Furaldehyd, Cellulose und Lignin aus Lignocellulosematerialien

Die Erfindung betrifft ein Verfahren sowie eine Vorrichtung zur Herstellung von 2-Furaldehyd, Cellulose und Lignin aus Lignocellulosematerialien, insbesondere in der Landwirtschaft anfallenden Lignocellulosematerialien, z.B. Maiskolben, Stroh sowie anderen Abfallprodukten von einjährigen Pflanzen, ferner auch aus Holz und Holzabfällen.

Die wirtschaftliche Verwertung von Biomassen genießt seit langem erhebliches soziales, wirtschaftliches und energiepolitisches Interesse, insbesondere im Bereich der Erzeugung von Lebensmitteln und Chemikalien sowie in der Pharmazie und Landwirtschaft. Dabei konzentrieren sich die Forschungsaktivitäten sowohl auf die Sicherstellung einer zukünftigen Rohstoffbasis als auch auf die Entwicklung billigerer, technologisch wie energetisch weniger anspruchsvoller und auch ökologisch annehmbarer Herstellungstechnologien, die nach Möglichkeit ohne Abfälle durchführbar sind. Zu derartigen Problemen gehört die Hydrolyse des Lignocellulose-Komplexes sowie die Abtrennung und Gewinnung der drei Hauptkomponenten dieses Komplexes, nämlich der Hemicellulosen und deren Abbauprodukte 2-Furaldehyd, Cellulose und Lignin.

Zur Ausnützung von pflanzlichem Kohlenstoff in Form von Lignocellulosematerialien und zur Gewinnung der angestrebten Endprodukte wurden Verfahren zur alkalischen oder sauren Hydrolyse am meisten untersucht und angewandt.

Die Anwendung dieser Verfahren zur Verarbeitung von Lignocellulosematerialien ist jedoch sehr kostenaufwendig, da die angewandten Laugen bzw. Säuren zurückgewonnen bzw. neutralisiert werden müssen, was einen entsprechend großen Reagensverbrauch, hohe Investitionskosten sowie den Anfall erheblicher Mengen chemischer Abfallprodukte mit sich bringt.

Etwa gleichzeitig mit der Entwicklung von Verfahren zur Hydrolyse von Lignocellulosematerialien mit verdünnten Säuren wurden Verfahren entwickelt, bei denen konzentrierte bzw. sehr konzentrierte Säuren angewandt werden. So ist ein Verfahren bekannt, bei dem das mit 37-%iger Salzsäure befeuchtete Lignocellulosematerial der Gegenstromextraktion unterzogen wird. Dieses Verfahren läßt sich durch Verwendung von 42-%iger Chlorwasserstoffsäure und intensive Kühlung der Reaktoren noch weiter verbessern.

Nach einem anderen Verfahren wird zur Hydrolyse gasförmiger Chlorwasserstoff verwendet; dabei wurde jedoch festgestellt, daß die angestrebte Hydrolyse erst bei einer Konzentration von Chlorwasserstoff von mehr als 90 % erreicht wird. Der gesamte Vorgang ist dabei in drei Stufen aufgeteilt:

Die Absorption von Chlorwasserstoff, die Hydrolyse sowie die Desorption des Chlorwasserstoffs. Schwierigkeiten dieser Verfahrensweise bestehen insbesondere darin, daß bei dem Kontakt von Chlorwasserstoff mit feuchtem Lignocellulosematerial erhebliche Wärmemengen freigesetzt werden, wodurch das hydrolysierte Material eine ungeeignete Konsistenz erhält und sich insbesondere nicht mehr für eine Verarbeitung in Fluidschichten bzw. Wirbelschichten eignet. Auf der anderen Seite werden ungenügend feuchte Rohstoffe mit nur geringer Ausbeute hydrolysiert. Die Problematik der Hydrolyse von Lignocellulosematerialien wurde daher in zahlreichen experimentellen Arbeiten untersucht, um optimale Bedingungen für die Hydrolyse mit Chlorwasserstoff aufzufinden, insbesondere im Hinblick auf die Aufbereitung des Rohmaterials und die Befeuchtung während des Prozesses. Nachteile der Anwendung von gasförmigem Chlorwasserstoff bestehen in den auftretenden Verlusten sowie dem Schwund und in der erheblichen Korrosionswirkung, die aufwendige Maßnahmen und die Verwendung teurer Reaktormaterialien erfordert. Die Korrosion beeinflußt entsprechend die Auswahl des Hydrolysereagens.

Darüber hinaus müssen bei der Mehrzahl der bisher bekannten Verfahren zur Hydrolyse von Lignocellulosematerialien noch Nachhydrolysierungsschritte durchgeführt werden. Aufgrund dieser technischen Schwierigkeiten und der Unwirtschaftlichkeit dieser Verfahren konnte bisher keine Technologie für die komplexe Nutzung der Produkte aus Pflanzen- und Holzmassen angegeben werden, die sowohl in technologischer als auch in wirtschaftlicher und ökologischer Hinsicht auch nur einigermaßen zufriedenstellend anwendbar ist.

Lignocellulosematerialien können auch durch Anwendung von Mikroorganismen oder mit Enzymen unter Dekristallisation und Hydrolyse aufgeschlossen werden. Auch diese Verfahren eignen sich bisher nicht für eine wirtschaftliche Nutzung von Biomassen, insbesondere nicht für den Aufschluß von Lignocellulosematerialien.

Außer von der angewandten Säure hängt der Verlauf der Hydrolyse von Lignocellulosematerialien in erheblichem Maße auch von den weiteren Bestandteilen des Reaktionsmediums ab. Die bisher bekannten Herstellungsverfahren für 2-Furaldehyd (Furfural) sind überwiegend einstufig; die Hydrolyse der Pentosane sowie die Dehydratation der Pentosen wird in Druckanlagen bei einem Druck von 0,7 bis 1,8 MPa durchgeführt; dabei werden ggfs. Katalysatoren, meistens Mineralsäuren, eingesetzt, wobei die im Prozeß freigesetzten Säuren, vor allem Essigsäure und Ameisensäure, als Katalysatoren wirken.

Alle bekannten Verfahrensweisen sind mit einer Reihe von Nachteilen behaftet, vor allem den folgenden:
- Die Ausbeute an 2-Furaldehyd erreicht nicht einmal 50 %, bezogen auf den Pentosan-Gehalt;
- die angewandten Druckreaktoren sind sowohl hinsichtlich der zu verwendenden Materialien als auch hinsichtlich ihrer Konstruktion aufwendig und arbeiten diskontinierlich; Dosierung von Reaktanten sowie die Entleerung solcher Reaktoren sind apparativ anspruchsvoll; die Reaktorgröße unterliegt erheblichen Einschränkungen, was sich wiederum in hohen Investitionskosten wie auch in hohen Betriebskosten widerspiegelt;
- eine Rückführung und Ausnützung der Abfallwärme ist bisher nicht möglich; die bekannten Verfahren sind mit einem hohen Energieverbrauch verbunden;
- die Konzentration von 2-Furaldehyd in den anfallenden Kondensaten ist verhältnismäßig gering, weshalb für seine Isolierung erhebliche Wärmemengen aufgewendet werden müssen;
- der Lignocellulose-Abfall weist einen beträchtlichen Feuchtigkeitsgehalt auf; er konnte bisher nicht in zufriedenstellender Weise genutzt werden; bei Verfahren, bei denen der Lignocellulose-Abfall trockener anfällt, wird er lediglich als Energiequelle durch Verbrennung verwendet;
- in einem drucklosen Fluidsystem können nur schütt- bzw. rieselfähige, partikelförmige pflanzliche Materialien verarbeitet werden. Feiner Flugstaub aus nichtklassierten Rohmaterialien verstopft, zusammen mit Kondensationsprodukten von 2-Furaldehyd, allmählich bestimmte Teile der Vorrichtungen, was einen kontinuierlichen Betrieb erschwert und die Betriebsfähigkeit beeinträchtigt. Eine Verminderung der Kondensation von 2-Furaldehyd in einem Inertgasstrom oder das Eindringen von Luftsauerstoff in die Vorrichtungen beim Eindosieren des partikelförmigen Ausgangsmaterials verringern die Ausbeute an 2-Furaldehyd;
- die in sehr hoher Verdünnung anfallenden Lösungen der bisher bekannten Prozesse sind nur unter hohem Energieaufwand aufzuarbeiten;
- die Anwendung von Mineralsäuren bei den bisher bekannten Prozessen erschwert die Abtrennung von 2-Furaldehyd aus dem Reaktionsgemisch;
- ein Nachteil des aus Abfällen bei der industriellen Delignifizierung isolierten Lignins besteht darin, daß es nur geringe Löslichkeit aufweist, strukturell erheblich verändert und mit Delignifizierungschemikalien verunreinigt ist;
- weitere Nachteile bei der Gewinnung von Lignin durch herkömmliche Hydrolyseprozesse liegen im hohen Kondensationsgrad des erhaltenen Lignins, in der niedrigen Ausbeute sowie dem Vorliegen von gelöster Hemicellulose-Komponente als Begleitmaterial in den isolierten Ligninen in einer Menge von 10 bis 20 %.

Der Erfindung liegt die Aufgabe zugrunde, ein wirksames, technisch einfaches und wirtschaftlich arbeitendes Verfahren zur Gewinnung von 2-Furaldehyd, Cellulose und Lignin zur Nutzung entsprechender Phytomassen, insbesondere in der Landwirtschaft, der Lebensmittelindustrie, der Pharmazie sowie der Chemie, und eine entsprechende Vorrichtung hierfür anzugeben, bei denen die oben erläuterten Nachteile des Stands der Technik vermieden sind; die Gewinnung der drei angestrebten Produkte soll dabei kontinuierlich und abfallfrei in einem thermischen Drucksystem durch Hydrolyse ohne Verwendung von Alkalien oder Säuren durchgeführt werden und hohe Ausbeuten an 2-Furaldehyd, Cellulose und Lignin ermöglichen.

Die Aufgabe wird anspruchsgemäß gelöst.

Die Unteransprüche betreffen vorteilhafte Ausführungsformen der Erfindungskonzeption.

Das erfindungsgemäße Verfahren zur kontinuierlichen Herstellung von 2-Furaldehyd, Cellulose und Lignin aus Lignocellulosematerialien ist gekennzeichnet durch folgende Schritte:

(A) Mischen des auf eine Teilchengröße von etwa 2 bis etwa 10 mm zerkleinerten Lignocellulosematerials mit Wasser in einem Massenverhältnis Lignocellulosematerial/Wasser von 1:5 bis 1:10 zu einer Suspension,

(B) Quellung der Suspension bei 70 bis 90 °C,

(C) Abpressen eines Teils des Wassers aus der Suspension,

(D) erste kontinuierliche Hydrolyse der gequollenen Suspension unter Druck bei 115 bis 135 °C,

(E) Abpressen der Flüssigkeit an der Suspension,

(F) zweite kontinierliche Hydrolyse unter Druck
- in einer ersten Phase bei 160 bis 180 °C und anschließend
- in einer zweiten Phase bei 200 bis 235 °C

(G) Abpressen der die Hauptmenge 2-Furaldehyd enthaltenden Flüssigkeit aus der Suspension und Gewinnung des 2-Furaldehyds,

(H) Druckabsenkung auf Atmosphärendruck und

(I) Extraktion des zerfallenen, unlöslichen Lignocelluloserestmaterials unter Gewinnung von Lignin und Cellulose.

Die Quellung in Stufe B wird vorteilhaft während einer Zeitdauer von 20 bis 180 min durchgeführt. Die erste kontinuierliche Hydrolyse in Stufe D erfolgt vorteilhaft während 10 bis 20 min, während die erste Phase der zweiten kontinuierlichen Hydrolyse in Stufe F vorzugsweise 10 bis 15 min und die zweite Phase der zweiten kontinuierlichen Hydrolyse in Stufe F vorzugsweise 0,5 bis 3 min durchgeführt werden.

Die erfindungsgemäße Vorrichtung zur kontinuierlichen Herstellung von 2-Furaldehyd, Cellulose und Lignin aus Lignocellulosematerialien, die sich insbesondere zur Durchführung des obigen Verfahrens eignet, ist gekennzeichnet durch

- einen Speicherbehälter für trockenes Lignocellulosematerial,
- einen auf mindestens 70 bis 90 °C beheizbaren Suspensionsvorratsbehälter, der vom Speicherbehälter beschickbar und mit einem Wasserspeicher verbunden ist und zur Quellung des darin eingeführten Lignocellulosematerials dient,
- eine Füllpresse, die mit dem unteren Teil des Suspensionsvorratsbehälters verbunden ist und zum Abpressen eines Teils des Wassers aus der Suspension des Lignocellulosematerials dient und ggfs. eine Rückführeinrichtung zur Rückführung der abgepreßten Flüssigkeit in den Suspensionsvorratsbehälter aufweist,
- einen mit dem Ausgang der Füllpresse verbundenen ersten kontinuierlichen, unter Druck arbeitenden Hydrolysereaktor mit einer Einrichtung zum Einführen von Flüssigkeit unter Druck zur Beheizung und Druckerzeugung,
- eine mit dem Ausgang des Hydrolysereaktors verbundene Überführungspresse zur Abtrennung von Flüssigkeit aus der Suspension aus dem Hydrolysereaktor und Überführung der abgepreßten Suspension sowie der abgetrennten Flüssigkeit in den nachgeschalteten Prozeß,
- einen mit dem Ausgang der Überführungspresse verbundenen zweiten kontinuierlichen Hydrolysereaktor mit zwei Einrichtungen zum Einführen der von der Überführungspresse abgetrennten Flüssigkeit unter Druck zur Beheizung und Druckerzeugung, wobei die erste Einrichtung am Eingang des Hydrolysereaktors unmittelbar nach dem Ausgang der Überführungspresse und die zweite Einrichtung in Strömungsrichtung danach, vorzugsweise im Bereich zwischen Mitte und Ende des Hydrolysereaktors, angeordnet sind,
- eine mit dem Ausgang des zweiten Hydrolysereaktors verbundene Ausdrückpresse zur Abtrennung von Flüssigkeit aus der Suspension aus dem zweiten Hydrolysereaktor und Überführung der abgepreßten Suspension in den nachgeschalteten Prozeß,
- eine mit dem Ausgang der Ausdrückpresse verbundene Entspannungseinrichtung zur Entspannung der eingeführten Suspension des aufgeschlossenen Lignocellulosematerials auf Atmosphärendruck mit einer Dampfabführungseinrichtung,
- eine mit dem Ausgang der Entspannungseinrichtung verbundene Extraktionsvorrichtung zur Extraktion des Lignins aus der eingeführten Suspension und Gewinnung des Lignins und der Cellulose,
- eine unter Atmosphärendruck arbeitende erste Rektifizierkolonne, deren Eingang mit dem Flüssigkeitsauslaß der Ausdrückpresse sowie mit der Dampfabführungseinrichtung der Entspannungseinrichtung verbunden ist,
- eine über einen Kondensator mit dem Kopf der Rektifizierkolonne verbundene Abscheidevorrichtung für 2-Furaldehyd zur Phasentrennung einer 2-Furaldehyd- und einer Wasserphase
sowie
- eine mit dem Ausgang für 2-Furaldehyd der Abscheidevorrichtung verbundene zweite Rektifizierkolonne, deren Kopfteil über einen Kondensator mit dem Eingang der Abscheidevorrichtung und deren Auslaß mit einem Speicher für 2-Furaldehyd verbunden ist.

Die Druckflüssigkeit mit dem Hauptanteil des 2-Furaldehyds durchläuft nach teilweiser Abkühlung die erste Rektifizierkolonne, in der eine azeotrope Rektifizierung des 2-Furaldehyds stattfindet und die durch Expansion entstandenen, mit 2-Furaldehyd angereicherten Dämpfe kondensiert werden; das Kondensat wird in der Abscheidevorrichtung, die ähnlich wie ein Scheidetrichter arbeitet, in eine 2-Furaldehyd- und eine Wasserphase aufgetrennt, wobei die 2-Furaldehydphase in der nachgeschalteten zweiten Rektifizierkolonne aufkonzentriert und der 2-Furaldehyd nachgereinigt wird.

Dabei ist vorteilhaft, wenn in die erste Rektifizierkolonne neben der von der Ausdrückpresse kommenden Druckflüssigkeit mit dem Hauptanteil des 2-Furaldehyds auch der Dampf von der Entspannungseinrichtung, der einen Restgehalt an 2-Furaldehyd enthält und bei der Expansion auf Atmosphärendruck entsteht, in die erste Rektifizierkolonne eingeführt wird.

Erfindungsgemäß ist es ferner vorteilhaft, den nach der Entspannung auf Atmosphärendruck vorliegenden, zerfallenen unlöslichen Lignocelluloserest mit einem Extraktionsmittel zu extrahieren, vorteilhaft mit einer wässerigen oder wässerig-ethanolischen Ammoniaklösung und/oder mit Aceton.

Nach einer vorteilhaften Ausführungsform der Erfindung wird die in den zweiten Hydrolysereaktor eingeführte Flüssigkeit, die auch zur Beheizung und zur Druckerzeugung dient, von der Überführungspresse zum zweiten Hydrolysereaktor durch einen Wärmeaustauscher hindurchgeleitet, in dem sie auf 130 bis 144 °C erhitzt wird; dieser Wärmeaustauscher wird vorteilhaft mit der Flüssigkeit betrieben, die aus der Ausdrückpresse nach dem zweiten Hydrolysereaktor austritt und die, da sie die Hauptmenge an 2-Furaldehyd enthält, in die erste Rektifizierkolonne eingeleitet wird; die in den zweiten kontinuierlichen Hydrolysereaktor eingeleitete Flüssigkeit wird ferner nach einer bevorzugten Ausführungsform in einer Heizeinrichtung nacherhitzt, in der zugleich die Aufteilung in die beiden Teilströme erfolgt. Im zweiten Hydrolysereaktor findet entsprechend eine zweistufige Hydrolyse statt.

## Beispiel

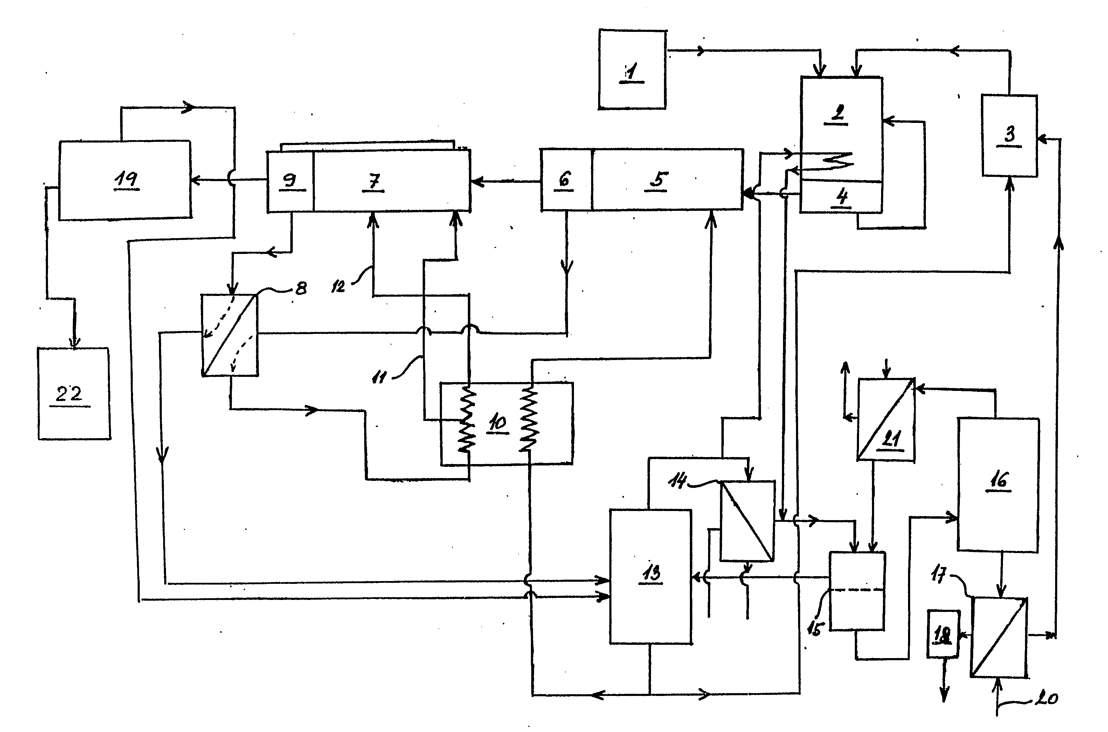

Die Durchführung des erfindungsgemäßen Verfahrens wird im folgenden unter Bezug auf eine vorteilhafte Ausführungsform der erfindungsgemäßen Vorrichtung anhand der Zeichnung näher erläutert.

Bei dem kontinuierlich durchgeführten Prozeß werden 500 kg Eingangs-Trockensubstanz/h durchgesetzt.

Das lose gelagerte Lignocellulose-Grundmaterial, d.h. der eingesetzte Rohstoff, z.B. Maiskolben, Stroh, udgl., wird, beispielsweise mit einem Greiferkran, zu einer Zuführeinrichtung befördert, an deren Austritt sich ein magnetischer Separator zur Abtrennung eventuell vorhandener unerwünschter ferromagnetischer Beimischungen befindet, und von dort mit einer Transporteinrichtung zu einer Schneidemaschine und Schlagmühle gefördert, wo die Eingangs-Trockensubstanz auf eine Partikelgröße von etwa 2 bis etwa 10 mm zerkleinert wird. Dieses Material wird weiter pneumatisch in den Speicherbehälter 1 gefördert, von wo es kontinuierlich in einer Menge von 500 kg/h in den Suspensionsvorratsbehälter 2 eingebracht wird, in den aus dem Wasserspeicher 3 Industriewasser in einer Menge von 250 kg/h zugeführt wird; dabei liegt ein Massenverhältnis von Lignocellulosematerial/Wasser von 1:7 bis 1:10 vor.

Die erhaltene Suspension wird mit Abdampf auf eine Temperatur von 70 bis 90 °C bei einer Verweilzeit von mindestens 20 bis 180 min erwärmt, wobei es zu einem Quellen der Trockensubstanz kommt. Anschließend wird die gequollene Suspension aus dem unteren Teil des Suspensionsvorratsbehälters 2 in die Füllpresse 4 gefördert, in der Wasser in einer Menge von etwa 4700 kg/h abgepreßt wird, das in den Suspensionsvorratsbehälter 2 umgepumpt wird. Das suspendierte Lignocellulosematerial gelangt dann durch die Füllpresse 4 in Form eines Suspensionspfropfens bei einem Massenverhältnis Lignocellulosematerial/Wasser von 1:0,6 bis 1:1 in einem Durchsatz von 800 kg/h, wovon 500 kg/h Trockensubstanz und 300 kg/h Wasser sind, in den ersten kontinuierlich betriebenen Hydrolysereaktor 5, in dem die Suspension durch Einspritzen einer auch zur Beheizung und Druckerzeugung dienenden Flüssigkeit, insbesondere von der ersten Rektifizierkolonne rückgeführter wässeriger Phase, mit einer Temperatur von 115 bis 150 °C, im Beispielsfalle 146 °C, kontinuierlich auf einer Hydrolysetemperatur von 130 °C gehalten wird; hierbei liegt ein Massenverhältnis Lignocellulosematerial/Wasser von 1:1,5 bis 1:3 bei einem Massendurchsatz von 1200 kg/h vor. Nach Durchlauf der Suspension durch den ersten Hydrolysereaktor 5 bei einer Verweilzeit von 10 bis 20 min geht die Suspension durch die Überführungspresse 6 in Form eines Suspensionspfropfens in den zweiten kontinuierlichen Hydrolysereaktor 7 über, und zwar bei einem Durchsatz von 635 kg/h, wovon 397 kg/h auf Trockensubstanz, 221 kg/h auf Wasser und 17 kg/h auf Xylose entfallen. In der Überführungspresse 6 wird ferner flüssige Phase in einer Menge von 1365 kg/h abgepreßt, wovon 1265 kg/h auf Wasser und 100 kg/h auf Xylose entfallen. Diese abgepreßte Flüssigkeit läuft durch den ersten Wärmeaustauscher 8 hindurch, in dem sie durch die Flüssigkeit von 130 auf 144 °C erwärmt wird, die mit der Ausdrückpresse 9 am Ausgang des zweiten Hydrolysereaktors 7 aus dem Hydrolysegemisch abgepreßt wird.

Aus dem ersten Wärmeaustauscher 8 gelangt die Flüssigkeit in eine Heizeinrichtung 10, die als Röhrenofen ausgebildet ist und in der sie zur Einspritzung in den zweiten Hydrolysereaktor 7 nacherhitzt wird. In der Heizeinrichtung 10 erfolgt zugleich die Aufteilung in zwei Teilströme 11 und 12, wobei der Teilstrom 11 eine Temperatur von 200 °C besitzt und ein Durchsatz von 910 kg/h vorliegt; der Teilstrom 12 besitzt eine Temperatur von 230 °C bei einem Durchsatz von 455 kg/h. Der Teilstrom 11 wird in den Suspensionspfropfen hinter dem Austritt der Überführungspresse 6 in den Hydrolysereaktor 7 eingeführt, während der Teilstrom 12 in einer Position eingeführt wird, der näher zur Ausdrückpresse 9 liegt. Bei dieser zweiten Hydrolyse liegt ein Massenverhältnis Lignocellulosematerial/Wasser von 1:1,5 bis 1:1,3 vor.

Im zweiten kontinuierlichen Hydrolysereaktor 7 wird der Suspensionspfropfen zerstört und die Suspension im ersten Teil durch Einspritzen des Teilstroms 11 der auch zur Beheizung und Druckerzeugung dienenden Flüssigkeit bei einer Verweilzeit von 10 bis 18 min auf 150 bis 180 °C erwärmt; beim Durchlaufen des anschließenden Teils des Hydrolysereaktors 7 wird die Suspension durch den eingespritzten zweiten Teilstrom 12 bei einer Verweilzeit von 0,5 bis 3 min auf 200 bis 235 °C erwärmt. Gleichzeitig wird dem zweiten Hydrolysereaktor 7 Wärme aus einer (nicht dargestellten) externen Quelle zugeführt.

Aus dem zweiten kontinuierlich betriebenen Hydrolysereaktor 7 gelangt die Suspension in die Ausdrückpresse 9, in deren erstem Teil die bereits erwähnte flüssige Phase einer Temperatur von 230 °C in einer Menge von 1365 kg/h abgepreßt wird, wovon 1301 kg/h auf Wasser und 64 kg/h auf 2-Furaldehyd entfallen. Die Flüssigkeit wird dann im Wärmeaustauscher 8 auf 190 bis 205 °C abgekühlt und zur unter Atmosphärendruck arbeitenden ersten Rektifizierkolonne 13 geleitet, die völlig durch Eigenwärme erwärmt wird. In der ersten Rektifizierkolonne entsteht Dampf einer Temperatur von 100 °C, ggfs. bis zu 80 °C, durch die Expansion; die abgekühlten Dämpfe, die in der Rektifizierkolonne 13 mit 2-Furaldehyd angereichert wurden, gelangen über ihren Kopfteil zum ersten Kondensator 14 in einer Menge von 375 kg/h, wovon 300 kg/h auf Wasser und 73 kg/h auf 2-Furaldehyd entfallen. Das Kondensat gelangt in der angegebenen Menge, ggfs. über einen Kühler, zu der Abscheidevorrichtung 15 für 2-Furaldehyd, die scheidetrichterartig ausgebildet ist und wo es zu einer Trennung der 2-Furaldehydphase von der Wasserphase kommt. Der 2-Furaldehyd wird aus der Abscheidevorrichtung 15 in eine zweite Rektifizierkolonne 16 eingeführt, die von außen beheizt wird und in der der 2-Furaldehyd aufkonzentriert und nachgereinigt wird. Das Konzentrat gelangt in einen mit Kühlwasser 20 gekühlten Wärmeaustauscher 17, dessen Wasserauslaß mit dem Wasserspeicher 3 verbunden ist, und tritt von dort in den Speicher 18 für 2-Furaldehyd ein. Zwischen der Rektifizierkolonne 16 und der Abscheidevorrichtung 15 ist ein zweiter Kondensator 21 vorgesehen, der, ebenso wie der erste Kondensator 14, an ein (nicht dargestelltes) Kühlsystem angeschlossen ist. Der Auslaß des Kondensators 21 ist an die Abscheidevorrichtung 15 angeschlossen.

Der unlösliche Lignocelluloserest, der aus der Ausdrückpresse 9 in Pfropfenform austritt, gelangt von dort in die Entspannungsvorrichtung 19, wo unter Expansion Entspannung auf Atmosphärendruck eintritt. Dabei zerfällt das Lignocellulosematerial. Der expandierte Dampf wird über eine (nicht dargestellte) Leitung über den Suspensionsvorratsbehälter 2 zur ersten Rektifizierkolonne 13 geleitet, um auch den darin enthaltenen Gehalt an 2-Furaldehyd dadurch zu gewinnen.

Der zerfallene, unlösliche Lignocelluloserest wird dann aus der Entspannungsvorrichtung 19 in eine Extraktionsvorrichtung 22 übergeführt, in der das Lignin extrahiert und damit Lignin und Cellulose gewonnen werden. Als Extraktionsmittel werden wässerige oder wässerig-ethanolische Ammoniaklösungen bzw. Aceton verwendet; die Extraktion erfolgt bei Temperaturen von 15 bis 40 °C.

Das Massenverhältnis Lignocellulosematerial/Wasser liegt in beiden Hydrolysereaktoren im Bereich von 1:3. Der Wasserspeicher 3 wird mit Wasser aus dem zweiten Wärmeaustauscher 17 gefüllt. Er ist ferner mit dem Flüssigkeitsauslaß der ersten Rektifizierkolonne 13 verbunden, der über die Heizeinrichtung 10 auch mit dem Flüssigkeitseinlaß des ersten Hydrolysereaktors 5 verbunden ist.

Auf die beschriebene Weise ist es möglich, hohe Ausbeuten der angestrebten Produkte zu erreichen:
- 2-Furaldehyd mit einer Ausbeute von etwa 15 %, bezogen auf die Masse an Eingangs-Trockensubstanz des Lignocellulosematerials, was 55 bis 62 % der theoretischen Ausbeute entspricht, d.h. etwa 150 kg/t;
- Cellulose in einer Ausbeute von 80 bis 90 % der theoretischen Ausbeute, d.h. etwa 330 kg/t;
- Lignin in einer Ausbeute von 75 bis 90 % der theoretischen Ausbeute, d.h. in einer Menge von etwa 130 kg/t.

Die Erfindungskonzeption führt zu einer bedeutenden Senkung der Betriebskosten bzw. der auf die Masse der Eingangs-Trockensubstanz bezogenen Verarbeitungskosten im Vergleich mit herkömmlichen Systemen um 35 bis 55 %, wobei zugleich höhere Produktqualität erzielt wird. Das Verfahren und die Vorrichtung arbeiten ferner aus ökologischer Sicht völlig umweltfreundlich, da keine Abfallprodukte entstehen.

Weitere Vorteile der Erfindungskonzeption sind darin zu sehen, daß die bisherigen, mit zahlreichen Nachteilen behafteten katalytischen Systeme bei der Herstellung von 2-Furaldehyd aus Lignocellulosematerialien, d.h. insbesondere Mineralsäuren, die in allen bisherigen Prozessen angewandt wurden, durch ein kombiniertes Wärme-Druckhydroly sesystem ersetzt sind, bei dem eine höhere Hydrolysetemperatur und ein höherer Druck bei zugleich kurzen Verweilzeiten die hydrolytische und dehydratisierende Wirkung der Mineralsäuren ersetzen.

Das erfindungsgemäße, in wässerigem Milieu arbeitende kontinuierliche System erleichtert ferner die Abtrennung des 2-Furaldehyds aus dem Reaktionsgemisch unter Erzielung höherer Ausbeuten als im Stand der Technik, wobei zugleich ein geringeres Massenverhältnis (Hydromodul) Lignocellulosematerial/Wasser aufgrund der kurzen Verweilzeiten in den beiden Hydrolysereaktoren ermöglicht wird, die auch durch die hohe Temperatur von bis zu 235 °C erzielt wird. Ferner ist die Abtrennung des 2-Furaldehyds energetisch wesentlich günstiger als bei bisherigen Verfahren.

Das erfindungsgemäße Verfahren ermöglicht gegenüber herkömmlichen Systemen mit Katalyse durch Einwirkung von Mineralsäuren die Gewinnung von reaktivem, unkondensiertem Lignin, das zur Herstellung von Kunstharzen, Kunststoffen sowie als aktives Additiv bei der Herstellung von Kautschuk und Gummi vorteilhaft verwendet werden kann.

Das reaktive Lignin ist in organischen Lösungsmitteln gut löslich und kann leicht in hoher Reinheit von den übrigen Komponenten des Reaktionsgemischs abgetrennt werden. Die anfallende Cellulose kann ferner als sehr reine, energiehaltige Komponente beispielsweise zur Tierfütterung verwendet oder zu Zuckerlösungen weiterverarbeitet werden.

Das erfindungsgemäße Verfahren sowie die erfindungsgemäße Vorrichtung sind folglich in sehr breitem Maße anwendbar, wobei Gleiches auch für die erzielten Reaktionsprodukte gilt.

Die erfindungsgemäße Technologie läßt sich insbesondere im Bereich der Landwirtschaft bei der Verarbeitung pflanzlicher Abfallprodukte, d.h. insbesondere von Abfällen aus der Primärproduktion, wie etwa von Maiskolben, verschiedenen Arten von Stroh sowie anderen Abfallprodukten einjähriger Pflanzen, vorteilhaft anwenden. Durch die optimale Verarbeitung und Ausnützung der Hauptkomponenten von Lignocellulosematerialien sind landwirtschaftliche Nebenproduktionen möglich, mit denen die Effektivität der landwirtschaftlichen Produktion erheblich gesteigert werden kann. Gleiches gilt auch etwa für die Forstwirtschaft, wo Holzabfälle entsprechend aufgearbeitet werden können, ferner für Cellulosefabriken.

Der 2-Furaldehyd kann in breiter Weise verwendet werden, besonders auch in der Bauindustrie, bei der Herstellung von Furan-Plastbeton sowie der Herstellung von Fural-Bodenbelägen. In der Chemie sind Verwendungsmöglichkeiten bei der Herstellung von Herbiziden, Lacken, Kittmaterialien, Imprägnierungssystemen udgl. gegeben, im Maschinenbau bei der Herstellung von Gußkernen und Gußformen für Gießereizwecke, ferner in der pharmazeutischen Industrie, in der Petrochemie u.v.a.

Die resultierenden Hydrolysate können ferner vorteilhaft in der Futtermittelindustrie, z.B. in Kombination mit Futtereiweißmaterialien, in der mikrobiologischen Industrie, bei der Herstellung von Substraten udgl., sowie ferner in der pharmazeutischen Industrie bei der Herstellung reiner Zucker, reiner Glucose, von Xylose udgl., sowie in der Lebensmittelindustrie vorteilhaft verwendet werden.

**Ansprüche**

1. Verfahren zur kontinuierlichen Herstellung von 2-Furaldehyd, Cellulose und Lignin aus Lignocellulosematerialien, **gekennzeichnet** durch folgende Schritte:

(A) Mischen des auf eine Teilchengröße von etwa 2 bis etwa 10 mm zerkleinerten Lignocellulosematerials mit Wasser in einem Massenverhältnis Lignocellulosematerial/Wasser von 1:5 bis 1:10 zu einer Suspension,

(B) Quellung der Suspension bei 70 bis 90 °C,

(C) Abpressen eines Teils des Wassers aus der Suspension,

(D) erste kontinuierliche Hydrolyse der gequollenen Suspension unter Druck bei 115 bis 135 °C,

(E) Abpressen der Flüssigkeit an der Suspension,

(F) zweite kontinuierliche Hydrolyse unter Druck
- in einer ersten Phase bei 160 bis 180 °C und anschließend
- in einer zweiten Phase bei 200 bis 235 °C,

(G) Abpressen der die Hauptmenge 2-Furaldehyd enthaltenden Flüssigkeit aus der Suspension und Gewinnung des 2-Furaldehyds,

(H) Druckabsenkung des verbliebenen Lignocelluloserestmaterials auf Atmosphärendruck und

(I) Extraktion des zerfallenen, unlöslichen Lignocelluloserestmaterials unter Gewinnung von Lignin und Cellulose.

2. Verfahren nach Anspruch 1, dadurch gekennzeichnet, daß die Quellung in Stufe B 20 bis 180 min und/oder die erste kontinuierliche Hydrolyse in Stufe D 10 bis 20 min und/oder die erste Phase der zweiten kontinuierlichen Hydrolyse in Stufe F 10 bis 15 min und/oder die zweite Phase der zweiten kontinuierlichen Hydrolyse in Stufe F 0,5 bis 3 min durchgeführt werden.

3. Verfahren nach Anspruch 1 oder 2, dadurch gekennzeichnet, daß die Extraktion des Lignins in Stufe I mit wässeriger Ammoniaklösung, wässerigethanolischer Ammoniaklösung und/oder mit Aceton als Extraktionsmitteln durchgeführt wird.

4. Verfahren nach einem der Ansprüche 1 bis 3, dadurch gekennzeichnet, daß in Stufe G aus der abgepreßten Flüssigkeit 2-Furaldehyd durch azeotrope Rektifizierung, Abtrennung des 2-Furaldehyds aus dem erhaltenen Kondensat und ggfs. anschließende zweite Rektifizierung gewonnen wird.

5. Verfahren nach Anspruch 4, dadurch gekennzeichnet, daß die erste, azeotrope Rektifizierung in Stufe G unter Atmosphärendruck durchgeführt wird.

6. Verfahren nach Anspruch 4 oder 5, dadurch gekennzeichnet, daß der in Stufe H anfallende, 2-Furaldehyd enthaltende Dampf in die Stufe der ersten, azeotropen Rektifizierung eingeführt wird.

7. Verfahren nach einem der Ansprüche 1 bis 6, dadurch gekennzeichnet, daß die in Stufe E nach der ersten kontinuierlichen Hydrolyse erhaltene, unter Druck stehende Flüssigkeit unter Nacherhitzung in zwei Teilströme aufgeteilt wird, wobei ein Teilstrom mit einer niedrigeren Temperatur in die erste Phase der Stufe F und der andere Teilstrom mit gegenüber dem ersten Teilstrom höherer Temperatur in die zweite Phase der Stufe F der zweiten kontinuierlichen Hydrolyse eingeführt wird.

8. Verfahren nach Anspruch 7, dadurch gekennzeichnet, daß die in Stufe E nach der ersten kontinuierlichen Hydrolyse erhaltene Flüssigkeit zunächst in einem Wärmeaustauscher vorerhitzt wird, insbesondere auf 130 bis 144 °C, der durch die in Stufe G anfallende Flüssigkeit beheizt wird, und anschließend unter Aufteilung in die beiden Teilströme nacherhitzt wird, wobei insbesondere der erste Teilstrom auf etwa 200 °C und der zweite Teilstrom auf etwa 230 °C erhitzt werden.

9. Vorrichtung zur kontinuierlichen Herstellung von 2-Furaldehyd, Cellulose und Lignin aus Lignocellulosematerialien, insbesondere zur Durchführung des Verfahrens nach einem der Ansprüche 1 bis 8,
gekennzeichnet durch
- einen Speicherbehälter (1) für trockenes Lignocellulosematerial,
- einen auf mindestens 70 bis 90 °C beheizbaren Suspensionsvorratsbehälter (2), der vom Speicherbehälter (1) beschickbar und mit einem Wasserspeicher (3) verbunden ist und zur Quellung des darin eingeführten Lignocellulosematerials dient,
- eine Füllpresse (4), die mit dem unteren Teil des Suspensionsvorratsbehälters (2) verbunden ist und zum Abpressen eines Teils des Wassers aus der Suspension des Lignocellulosematerials dient und ggfs. eine Rückführeinrichtung zur Rückführung der abgepreßten Flüssigkeit in den Suspensionsvorratsbehälter (2) aufweist,
- einen mit dem Ausgang der Füllpresse (4) verbundenen ersten kontinuierlichen, unter Druck arbeitenden Hydrolysereaktor (5) mit einer Einrichtung zum Einführen von Flüssigkeit unter Druck zur Beheizung und Druckerzeugung,
- eine mit dem Ausgang des Hydrolysereaktors (5) verbundene Überführungspresse (6) zur Abtrennung von Flüssigkeit aus der Suspension aus dem Hydrolysereaktor (5) und Überführung der abgepreßten Suspension sowie der abgetrennten Flüssigkeit in den nachgeschalteten Prozeß,
- einen mit dem Ausgang der Überführungspresse (6) verbundenen zweiten kontinuierlichen Hydrolysereaktor (7) mit zwei Einrichtungen (11, 12) zum Einführen der von der Überführungspresse (6) abgetrennten Flüssigkeit unter Druck zur Beheizung und Druckerzeugung, wobei die erste Einrichtung (11) am Eingang des Hydrolysereaktors (7) unmittelbar nach dem Ausgang der Überführungspresse (6) und die zweite Einrichtung (12) in Strömungsrichtung danach, vorzugsweise im Bereich zwischen Mitte und Ende des Hydrolysereaktors (7), angeordnet sind,
- eine mit dem Ausgang des zweiten Hydrolysereaktors (7) verbundene Ausdrückpresse (9) zur Abtrennung von Flüssigkeit aus der Suspension aus dem zweiten Hydrolysereaktor (7) und Überführung der abgepreßten Suspension in den nachgeschalteten Prozeß,
- eine mit dem Ausgang der Ausdrückpresse (9) verbundene Entspannungseinrichtung (19) zur Entspannung der eingeführten Suspension des aufgeschlossenen Lignocellulosematerials auf Atmosphärendruck mit einer Dampfabführungseinrichtung,
- eine mit dem Ausgang der Entspannungseinrichtung (19) verbundene Extraktionsvorrichtung (22) zur Extraktion des Lignins aus der eingeführten Suspension und Gewinnung des Lignins und der Cellulose,
- eine unter Atmosphärendruck arbeitende erste Rektifizierkolonne (13), deren Eingang mit dem Flüssigkeitsauslaß der Ausdrückpresse (9) sowie mit der Dampfabführungseinrichtung der Entspannungseinrichtung (19) verbunden ist,
- eine über einen Kondensator (14) mit dem Kopf der Rektifizierkolonne (13) verbundene Abscheidevorrichtung (15) für 2-Furaldehyd zur Phasentrennung einer 2-Furaldehyd- und einer Wasserphase sowie
- eine mit dem Ausgang für 2-Furaldehyd der Abscheidevorrichtung (15) verbundene zweite Rektifizierkolonne (16), deren Kopfteil über einen Kondensator (21) mit dem Eingang der Abscheidevorrichtung (15) und deren Auslaß mit einem Speicher (18) für 2-Furaldehyd verbunden ist.

10. Vorrichtung nach Anspruch 9, gekennzeichnet durch einen Wärmeaustauscher (8), der beheizungsseitig zwischen den Flüssigkeitsauslaß der Ausdrückpresse (9) und den Eingang der ersten Rektifizierkolonne (13) geschaltet ist und der sekundärseitig zwischen den Flüssigkeitsauslaß der Überführungspresse (6) und die beiden Einrichtungen (11, 12) zum Einführen von Flüssigkeit des zweiten Hydrolysereaktors (7) geschaltet ist.

11. Vorrichtung nach Anspruch 10, gekennzeichnet durch eine Heizeinrichtung (10), die einerseits mit der Sekundärseite des Wärmeaustauschers (8) verbunden ist und zwei Ausgänge aufweist, die mit der ersten Einrichtung (11) bzw. der zweiten Einrichtung (12) zum Einführen von Flüssigkeit in den zweiten Hydrolysereaktor (7) verbunden sind und zur Nacherhitzung der vom Wärmeaustauscher (8) kommenden Flüssigkeit dient, und die andererseits zwischen den Flüssigkeitsauslaß der ersten Rektifizierkolonne (13) und den Flüssigkeitseinlaß des ersten Hydrolysereaktors (5) eingeschaltet ist.

EP 0 346 836 A2